# EUROPÄISCHE PATENTANMELDUNG

(11) **EP 1 564 648 A2**
(43) Veröffentlichungstag der Anmeldung: **17.08.2005**
(21) Anmeldenummer: 05002808.3
(22) Anmeldetag: 10.02.2005
(51) Int. Cl.: G06F 17/00, G01N 35/00

(54) **Verfahren zur Überwachung von Laborproben**

(30) Priorität: 14.02.2004 DE 102004007298
(71) Anmelder: Universitätsklinikum Düsseldorf Anstalt des öffentlichen Rechts, 40225 Düsseldorf (DE)
(72) Erfinder: Becker, Annette, 40239 Düsseldorf (DE)
(74) Vertreter: Christophersen, Ruth

(57) **Zusammenfassung**

Es wird ein Verfahren zur Überwachung von zu untersuchenden Proben beansprucht, die nach der Probenentnahme an einem vom Ort der Probenentnahme verschiedenen Ort untersucht werden, welches die folgenden Schritte umfasst:
a) Erfassen der Probe in dem Datensystem (DS) des Auftraggebers,
b) Generierung eines Laborauftrages aus den Daten über vorzunehmende Analysen/Untersuchungen,
c) Erstellung eines probenbezogenen Datensatzes im Datensystem (DS) des Auftraggebers und Übermittlung des Datensatzes an das Laborsystem (LS) des beauftragten Labors sowie an das Probenüberwachungssystem,
d) Zusammenstellen der für das beauftragte Labor bestimmten Proben zu mindestens einer Charge, im Datensystem (DS) des Auftraggebers Generierung eines für die Charge spezifischen Chargen-Identifizierungscodes, z.B. einer Fallnummer, sowie Zuordnung der Datensätze der einzelnen Proben der Charge zu dem Chargen-Identifizierungscode,
e)
   i. ggf. Generierung eines Transportauftrags an einen Transportdienst,
   ii. Erfassung der Abholung der Probe(n) bzw. Charge(n) im Probenüberwachungssystem durch einen Transportdienst und Verknüpfung des Auftrages mit den Chargen-Identifizierungscodes der zu transportierenden Chargen,
f) Transport der Proben an das beauftragte Labor,
g) per Datentransfer an das Datensystem (DS) des Auftraggebers Bestätigung über den Probeneingang im beauftragten Labor anhand des Chargen-Identifizierungscodes und/oder der Datensätze der einzelnen Proben,
h) Durchführung der Untersuchung,
i) per Datentransfer an das Datensystem (DS) des Auftraggebers Übermittlung der mit dem jeweiligen Datensatz verknüpften Untersuchungsergebnisse.

## Beschreibung

Die vorliegende Erfindung betrifft ein Verfahren zur Überwachung von zu untersuchenden Proben, die nach der Probenentnahme an einem vom Ort der Probenentnahme verschiedenen Ort untersucht werden.

Die Analyse von Proben, insbesondere von Patientenproben aus Krankenhäusem, Lebensmittelproben und Wasser- und Bodenproben aus Umwelt und Industrie erfolgt vielfach nicht vor Ort bzw. in den jeweiligen Krankenhäusern oder Industriebetrieben, sondem in Labors, die auf die Untersuchung dieser Proben spezialisiert sind. Diese Labors befinden sich in der Regel nicht im gleichen Gebäude, in dem die Probenentnahme stattfindet. Der Probentransport führt zwangsläufig zu einer zeitlichen Verzögerung der Untersuchung. Hinzu kommt, dass die Gefahr besteht, dass Proben verwechselt oder auch vergessen werden.

In Krankenhäusern sind die Untersuchungen von Blut, Urin etc. notwendig, um den Zustand des Patienten diagnostizieren zu können und um daraufhin eine konkrete Behandlung einzuleiten. Die für die Untersuchung erforderlichen Proben werden am Patienten durch das Stationspersonal entnommen (abgenommen) und in dafür vorgesehene Probenröhrchen oder andere geeignete Probenbehälter gefüllt. Anschließend werden die Proben in das Labor geschickt. Der Probentransport erfolgt in der Regel durch einen separaten Transportdienst. Im Labor werden die entsprechenden Untersuchungen durchgeführt, hierzu werden die Proben den jeweiligen Analysegeräten zur Bestimmung der angeforderten Parameter zugeordnet. Nach Abschluss der Analyse und nach Freigabe der Ergebnisse werden diese dem Auftraggeber, im Krankenhaus der anfordemden Station, übermittelt.

Die Identifizierung der Proben erfolgt üblicherweise durch einen so genannten Barcode, der als Informationen Auftraggeber, den Inhalt der Probe, die Art der Untersuchung und ggf. weitere Daten enthält.

In der skizzierten Ablaufkette gibt es viele Fehlerquellen, durch welche die zügige Untersuchung der Proben behindert wird. So kann es geschehen, dass vergessen wird, die Proben zu entnehmen oder nicht genug Material entnommen wird. Auch geschieht es, dass Identifizierungsetiketten unleserlich werden oder Proben auf dem Transport zum Labor verloren gehen. Bei einer Verschlechterung des Patientenzustandes werden Routineproben zu Notfallproben, deren Untersuchung umgehend durchgeführt werden muss. Bei dem in der Praxis üblichen Ablauf ist es jedoch nicht ohne weiteres möglich, bereits abgenommene Proben schnell aufzufinden und deren Untersuchung zu beschleunigen. Auch beim Einsatz eines elektronischen Anforderungsverfahrens gibt es keine Information über die erfolgte Abnahme, Vollständigkeit oder aktuellen Aufenthaltsort der Proben. Für den Anforderer der Untersuchung, in der Regel der Arzt, ist es nicht möglich, festzustellen, ob alle erforderlichen Proben korrekt abgenommen und auf den Weg gebracht wurden, wo sich die Proben derzeit befinden und ob diese schon untersucht werden. Erst am Ende eines Routinetages kann festgestellt werden, ob ggf. noch Ergebnisse fehlen. Durch jede einzelne Verzögerung im Ablauf der Untersuchung wird auch die Behandlung eines Patienten verzögert, was wiederum weitere Probleme nach sich ziehen kann.

Der vorliegenden Erfindung lag die Aufgabe zugrunde, ein Verfahren zur Überwachung von zu untersuchenden Proben zur Verfügung zu stellen, welches die voranstehend beschriebenen Nachteile nicht aufweist. Insbesondere für den Einsatz in Krankenhäusern ist es erwünscht, ein Verfahren zur Verfügung zu stellen, welches eine nach der Probenentnahme, bevorzugt bereits ab Entscheidung über die Durchführung einer Laboruntersuchung und Aufnahme der Daten in ein Datensystem, Überwachung der Proben ermöglicht. Die Zeit zwischen Probenentnahme und Eintreffen der Probe im Labor soll kurz und frei von logistischen Verzögerungen sein. Femer ist es wünschenswert, wenn der Auftraggeber und ggf. auch das bereits beauftragte Labor Kenntnis hat, wo in der üblichen Ablaufkette die Probe sich befindet bzw. zeitnah über Störungen im Ablauf informiert wird.

Gegenstand der vorliegenden Erfindung ist demgemäß ein Verfahren zur Überwachung von zu untersuchenden Proben, die nach der Probenentnahme an einem vom Ort der Probenentnahme verschiedenen Ort untersucht werden, welches die folgenden Schritte umfasst:
a) Erfassen der Probe in dem Datensystem (DS) des Auftraggebers,
b) Generierung eines Laborauftrages aus den Daten über vorzunehmende Analysen/Untersuchungen,
c) Erstellung eines probenbezogenen Datensatzes im Datensystem (DS) des Auftraggebers und Übermittlung des Datensatzes an das Laborsystem (LS) des beauftragten Labors sowie an das Probenüberwachungssystem,
d) Zusammenstellen der für das beauftragte Labor bestimmten Proben zu mindestens einer Charge, im Datensystem (DS) des Auftraggebers Generierung eines für die Charge spezifischen Chargen-Identifizierungscodes, z.B. einer Fallnummer, sowie Zuordnung der Datensätze der einzelnen Proben der Charge zu dem Chargen-Identifizierungscode,
e) i. ggf. Generierung eines Transportauftrags an einen Transportdienst, ii. Erfassung der Abholung der Probe(n) bzw. Charge(n) im Probenüberwachungssystem durch einen Transportdienst und Verknüpfung des Auftrages mit den Chargen-Identifizierungscodes der zu transportierenden Chargen,
f) Transport der Proben an das beauftragte Labor,
g) per Datentransfer an das Datensystem (DS) des Auftraggebers Bestätigung über den Probeneingang im beauftragten Labor anhand des Chargen-Identifizierungscodes und/oder der Datensätze der einzelnen Proben,
h) Durchführung der Untersuchung,
i) per Datentransfer an das Datensystem (DS) des Auftraggebers Übermittlung der mit dem jeweiligen Datensatz verknüpften Untersuchungsergebnisse.

Das erfindungsgemäße Überwachungssystem für Laborproben hat den Vorteil, dass mit der Probenentnahme und Aufnahme der Probe in ein Datensystem unmittelbar die Zuordnung der Probe zu Laboraufträgen und auch eine Proben-Vollständigkeitskontrolle vorgenommen werden kann. Gleichzeitig besteht die Möglichkeit, den Abtransport einer Probe oder mehrerer Proben zu optimieren und auch durch die frühzeitige Auftragserteilung an das Labor den dortigen Untersuchungsablauf vorzubereiten. Das gilt auch für die Analyse von Proben, deren Dringlichkeit nachträglich auf "Notfall" hochgesetzt wurde. Ein ständiger Abgleich der Datensätze einer Probe im System der Auftraggebers und im Laborsystem ist daher möglich.

Im Rahmen der vorliegenden Erfindung umfasst der Ausdruck Datensystem das System, in welches die Daten der Probe zur Erfassung und Überwachung der Probe eingegeben werden, dies können das Laborsystem oder das Probenüberwachungssystem selbst, aber auch ein beliebiges anderes elektronisches Anforderungssystem sein, das über eine Schnittstelle mit dem DV-System eines zentralen Dienstleisters kommuniziert. Das Datensystem kann z.B. eine Auftragsmaske des Laborsystems, eines Praxissystems, eines Krankenhausinformationssystems oder die Eingabemaske des Probenüberwachungssystems sein. Das Probenüberwachungssystem und das Laborsystem können als ein gemeinsames Datensystem ausgestaltet sein oder es kann sich um zwei von einander getrennt arbeitende, aber miteinander im Datenaustausch stehende Systeme handeln. In der zuletzt genannten Ausgestaltung ist es jedoch bevorzugt, dass die einzelnen Systeme derart miteinander verknüpft sind, dass der Auftraggeber über das Probenüberwachungssystem erkennen kann, ob und wann die Probe vom Laborsystem erfasst wurde und besonders bevorzugt, wann das Untersuchungsergebnis voraussichtlich vorliegen wird. Auch ist es bevorzugt, wenn über das Laborsystem der Status und der Ort, an welchem sich die Probe gerade befindet, überprüft werden kann.

Zur Durchführung des erfindungsgemäßen Verfahrens wird seitens des Auftraggebers entschieden, ob und in welcher Form die Probe entnommen wird und welche Untersuchung durchzuführen ist. Auftraggeber im Sinne der vorliegenden Erfindung wird bei der Untersuchung von Patientenproben in einem Krankenhaus der untersuchende und behandelnde Arzt sein, bei der Untersuchung von Lebensmittelproben diejenige Person, die über Art und Umfang der Untersuchung zu entscheiden hat, gleiches gilt für die Untersuchung im Rahmen der Umweltanalytik. Die Person, die im Rahmen der vorliegenden Erfindung Auftraggeber ist, kann gleichzeitig auch die Person sein, die die Proben entnimmt und/oder die Person, die entscheidet, welche Untersuchungen durchzuführen sind. Bereits mit der Entscheidung über die durchzuführende Analyse kann gemäß Schritt a) diese in das Datensystem aufgenommen werden und gemäß Schritt b) ein Laborauftrag erteilt werden.

In Schritt c) wird ein probenbezogener Datensatzes im Datensystem (DS) des Auftraggebers erstellt und dieser mit dem entsprechenden Auftrag über vorzunehmende Analysen/Untersuchungen an das Laborsystem (LS) des beauftragten Labors und an das Probenüberwachungssystem übermittelt. In einer möglichen Ausführungsform der vorliegenden Erfindung ist es möglich, bereits vor der eigentlichen Probennahme, den Auftrag einschließlich Art, Umfang und Dringlichkeit der Untersuchung an das Labor zu melden. Dieser Schritt kann aber auch nach der Probennahme erfolgen. Ob die Verfahrensschritte b) und c) ganz oder in Teilschritten vor, nach oder parallel zur Probenentnahme erfolgt, kann z. B. von den Organisationsabläufen beim Auftraggeben etc. abhängen.

Die Probenentnahme selbst erfolgt in an sich bekannter Weise. Sofern es sich um flüssige Probematerialien handelt, werden diese in einen geeigneten Probenbehälter abgefüllt. Sofern in der vorliegenden Anmeldung der Ausdruck "Probe" verwendet wird, ist damit die Probe ggf. abgefüllt in einem entsprechendem Probenbehälter gemeint. Liegt das Probenmaterial in fester und für die Umgebung toxikologisch unbedenklicher Form vor, so ist es auch möglich, dass die Probe als Stückgutprobe ohne in einem entsprechenden Probenbehälter abgefüllt zu sein vorliegt, oder in spezielle Probenbehälter gegeben wird, die in Größe und Form von den üblicherweise verwendeten standardisierten Probenbehältern abweichen können.

Erfindungsgemäß werden die Daten der entnommenen Proben, sofern nicht bereits vor der Probenentnahme geschehen, spätestens jetzt von einem Datensystem erfasst und ein Datensatz erstellt, vorzugsweise einem elektronischen Datensystem. In das Datensystem sollten vorzugsweise alle mit der Untersuchung der Probe zusammenhängenden Daten eingegeben werden, wie Auftraggeber, Herkunft der Probe (Patient, Wasser, Boden etc.), Anlass der Probenentnahme sowie Art und Umfang der durchzuführenden Untersuchungen sowie auch Datum und Uhrzeit der Probenentnahme. Für eine zügige Abwicklung der Untersuchung ist es vorteilhaft, wenn das Datensystem auch Angaben über den gewünschten Abschluss der Untersuchungen enthält. Die Aufnahme dieser Daten hat den Vorteil, dass, wenn der Untersuchungsauftrag gemäß Schritt b) an das Labor ergeht, das Labor gleichzeitig auch eine Mitteilung erhält, wie schnell die Untersuchung durchzuführen ist. Da es unter Umständen sehr aufwendig sein kann, für jede Probe den gewünschten Termin, bis zu welchem die Untersuchung abgeschlossen sein soll, einzugeben, hat es sich als geeignet erwiesen, die Probe mit einer so genannten Dringlichkeitsstufe zu klassifizieren. Aus dieser Klassifizierung entnimmt das untersuchende Labor die Information, wie schnell bzw. bis zu welchem Termin das Untersuchungsergebnis vorliegen soll.

In einer besonderen Ausgestaltung der vorliegenden Erfindung bestätigt das Labor nach der Auftragserteilung den Auftrag. Das Labor kann dem Auftraggeber auch Hinweise geben, in welcher Form und Menge die Probe für die Analyse vorliegen sollte und ob nach Probenentnahme eine besondere Behandlung des Probenmaterials erforderlich ist, beispielsweise die Zugabe von Reagenzien zum Probenmaterial, um Veränderungen des Materials zwischen der Probenentnahme und er Analyse zu vermeiden, oder Kühlen des Materials.

Zur Vereinfachung des weiteren Verfahrens und um die Möglichkeit zu erhalten, den Gang der Probe selbst auch über elektronische Medien verfolgen zu können, hat es sich als vorteilhaft erwiesen, die Proben jeweils mit einem maschinenlesbaren Identifizierungscode, vorzugsweise einem so genannten Barcode, zu versehen. Ein solcher Proben-Identifizierungscode wird vorzugsweise aus dem in das Datensystem, beispielsweise in das Laborsystem, eingegebenen Datensatz erstellt. Besonders bevorzugt ist dieser Identifizierungscode auch von solchen elektronischen Lesegeräten lesbar, wie sie vom Transportdienst und vom beauftragten Labor eingesetzt werden. Auf diese Weise ist es möglich, während der gesamten Laufzeit der Probe die einzelnen Stationen elektronisch zu erfassen und den jeweiligen Standort bzw. den Status der Probe abzufragen.

Die abgenommene, beauftragte und ggf. mit einem Identifizierungscode gekennzeichnete Probe wird in einem nächsten Schritt für den Transport in das Labor vorbereitet. Üblicherweise wird die mit dem Barcode gekennzeichnete Probe in einen Probensammler gegeben. In diesem Probensammler werden gemäß Schritt d) eine oder mehrere für das beauftragte Labor bestimmte Proben zu mindestens einer Charge zusammengestellt. Im Datensystem (DS) des Auftraggebers wird ein für die Charge spezifischer Chargen-Identifizierungscode generiert, ferner erfolgt die Zuordnung der Datensätze, oder falls vorhanden der Identifizierungscodes, der einzelnen Proben der Charge zu dem Chargen-Identifizierungscode. Im Chargen-Identifizierungscode werden vorzugsweise die Datensätze aller im Probensammler befindlichen Proben zusammengefasst. Es ist bevorzugt, dass die im Probensammler befindliche Probe(n) automatisch an einer Leseeinheit für den Identifizierungscode vorbeigeführt werden, um die Proben zu erfassen.

Wenn die Probe anhand ihres Codes erkannt und einem Laborauftrag zugeordnet werden kann, kann die die Probe in einem nächsten Zwischenschritt im Überwachungssystem "als für den Transport zum Labor vorbereitet" erfasst werden und wird einem geeigneten Transportbehälter befördert. Der Transportbehälter ist vorzugsweise ebenfalls mit einer elektronisch lesbaren Kennzeichnung versehen.

Durch Aufnahme der einzelnen Datensätze der erfassten Proben in das Probenüberwachungssystem, werden in Abhängigkeit von den vorgegebenen Bedingungen und Regeln in Schritt e) die entsprechenden Transportaufträge generiert. Die Transportaufträge werden mit den Chargen-Identifizierungscodes der zu transportierenden Chargen verknüpft. Die Probe kann jetzt gemäß Schritt f) zum Labor transportiert werden.

Aus ökonomischen Gründen ist es nicht sinnvoll, wenn der Transportdienst nach jeder einzelnen Probenerfassung beauftragt wird. Es ist derzeit übliche Praxis, dass die Transportdienst in regelmäßigen Abständen, einmal oder mehrmals täglich, unaufgefordert die Stationen der Krankenhäuser, Arztpraxen etc. aufsuchen, um etwaige Proben abzuholen. Vorzugsweise wird ein Transportauftrag bzw. ein Leerungsauftrag für den Transportbehälter dann automatisch vom Datensystem generiert und erteilt, wenn bestimmte Bedingungen gegeben sind. Beispiele für einen so genannten Leerungs- bzw. Transportauftrag können sein, wenn beispielsweise ein Notfallauftrag erteilt wird und Notfallprobe(n) vollständig im Datensystem erfasst sind, die älteste Probe seit xx Minuten im Datensystem erfasst ist, wobei die Zeit xx konfigurierbar ist nach beispielsweise Sammelstelle, Tageszeit und Kalenderdatum, der Transportbehälter zu yy Prozent gefüllt ist, wobei yy konfigurierbar ist nach beispielsweise Sammelstelle, Tageszeit und Kalenderdatum, oder der Leerungsauftrag aufgrund einer besonderen Notfallsituation aktiv vom Auftraggeber ausgelöst wird. Das Verfahren erlaubt aber auch eine spontane Abholung durch den Transportdienst und die Bereitstellung der entsprechenden Informationen und des Probenstatus im Probenüberwachungssystem.

In Fällen, in denen das Probenmaterial in spezielle Probenbehälter abgefüllt wird, die auf Grund ihrer Größe oder Form nicht in den Transportbehälter passen, wird diese Probe an einer separaten Leseeinheit des Probensammlers erfasst und für den Transport vorgesehen, der vorzugsweise jedoch mit der Abholung und dem Transport der üblichen Transportbehälter koordiniert wird.

Für den Fall, dass eine Probe vom Datensystem nicht identifiziert werden kann, wird vorzugsweise ein Behälter bzw. eine Ablage für so genannte "Schlechtproben" eingerichtet. Die nicht lesbare Probe wird in diesem Fach abgelegt und vorzugsweise erfolgt eine Meldung an das Datensystem. Vorzugsweise erfolgt regelmäßig ein Abgleich zwischen Datum und Uhrzeit der Probenerfassung bzw. Freigabe des Analysenauftrags und der Erfassung der Probe im Transportsystem und nachfolgend im Labor. Wird der Eingang der Probe nach einer Wartezeit zz, welche beliebig konfigurierbar ist, nicht vom Transportsystem oder im Labor festgestellt, gibt das System eine Fehlermeldung bzw. ein Warnsignal. Auf diese Weise wird vermieden, dass fehlende oder nicht-lesbare Proben vergessen werden. Dieses Signal bzw. die Fehlermeldung kann beispielsweise dann ausgelöst werden, wenn die Anzahl dieser Schlechtproben eine bestimmte Zahl überschreitet oder nach Ablauf des letzten Einwurfs einer nicht lesbaren Probe eine bestimmte Zeit überschritten ist. Auch ist es bevorzugt, wenn die Auftraggeber für die Proben die Möglichkeit haben, die für nicht lesbare Proben vorgesehenen Behälter selbst entleeren zu können, um ggf. die nicht lesbare Probe durch eine neue Probe zu ersetzen oder mit einem neuen lesbaren Identifizierungscodes zu versehen, so dass die Probe dann den ordnungsgemäßen Probenablauf durchlaufen kann.

Zusammenfassend sollten die Proben, die in den Probensammler abgelegt wurden, spätestens nach einer vorbestimmten Zeit T zum Labor transportiert werden, wobei die vorbestimmte Zeit T auch von der Dringlichkeitsstufe abhängen kann.

In einer bevorzugten Ausführungsform der vorliegenden Erfindung wird aufgrund der an den Transportdienst ergangenen Leerungsaufträge vom Überwachungsdatensystem eine laufend aktualisierte Abholliste erzeugt. Es ist auch möglich, dass die Leerungsaufträge von einem vom Überwachungsdatensystem getrennten Modul, wie z.B. einer Transportplanungssoftware, verarbeitet werden (Datenübergabe, Datentransfer). Es ist bevorzugt, dass die Transportaufträge und der Status der Aufträge auch von dem Datensystem, welches die Proben erfasst, angezeigt und somit überwacht werden können.

Zur Abholung der Proben ist es bevorzugt, wenn sich der beauftragte Transportdienst mit entsprechenden Berechtigungscodes ausweisen kann. Besonders bevorzugt sollte der Transportdienst nur aufgrund des Berechtigungscodes befähigt sein, den Transportbehälter mit den entsprechenden Proben abzuholen. Mit der Identifizierung gegenüber dem Transportbehälter und der Entnahme dieses Behälters kann gleichzeitig eine entsprechende Mitteilung an das Überwachungssystem ergehen, dass der bei der Entnahme verriegelte Behälter und damit die darin erfassten Proben zum Labor auf den Weg gebracht wurden. Mit der Abholung durch den Transportdienst erfolgt vorzugsweise eine Meldung über diesen Vorgang an das Datensystem des Auftraggebers und, wenn dieses unabhängig vom System des Labors betrieben wird, auch eine Meldung an das Labor.

Der verwendete Transportbehälter wird bei Ankunft im Labor, vorzugsweise ebenfalls über geeignete Lesesysteme, identifiziert und die erhaltenen Proben werden registriert bzw. sofern die Laboraufträge bereits angekommen sind, werden die eingegangenen Proben mit den entsprechenden Aufträgen abgeglichen. Vorzugsweise erfolgt gemäß Schritt g) seitens des Labors per Datentransfer an das Datensystem DS des Auftraggebers eine Bestätigung über den Eingang der Probe im beauftragten Labor anhand des Chargen-Identifizierungsscodes und/oder Identifizierungscodes der einzelnen Proben an den Auftraggeber und vorzugsweise auch an den Transportdienst. Auf diese Weise ist es möglich, ggf. fehlende oder fehlerhafte Proben unmittelbar zu identifizieren und ggf. eine Fehlermeldung an den Auftraggeber zu senden. Die erhaltenen Proben können mit Hilfe eines üblichen Sortierers und in Kombination mit den eingegangenen Aufträgen nach Probenmaterialien sortiert werden. Eine mögliche und in der vorliegenden Erfindung einsetzbare Vorrichtung zum automatischen Sortieren von mit Barcodes versehenen Probenröhrchen ist beispielsweise im deutschen Gebrauchsmuster 296 22 078 U1 offenbart.

Im Labor wird gemäß Verfahrensschritt h) die Untersuchung durchgeführt. Dazu kann es erforderlich sein, die Probe entsprechend vor- bzw. aufzubereiten. Im Anschluss daran erfolgt die eigentliche Untersuchung bzw. Messung in den entsprechenden Analysegeräten. Die Untersuchungsergebnisse werden, ggf. nach Ihrer gesonderten Freigabe durch den Laborleiter etc., per Datentransfer an das Datensystem (DS) des Auftraggebers übermittelt, wobei die jeweiligen Ergebnisse mit dem jeweiligen Proben-Identifizierungscode verknüpft sind (Schritt i).

Um auch am Probensammler bzw. am Transportbehälter erkennen zu können, ob noch Proben auf Abholung warten bzw. wann die letzte Probenleerung erfolgte, hat es sich als geeignet erwiesen, wenn der Behälter eine Anzeigevorrichtung aufweist, an der vorzugsweise Leerungszyklus bzw. Uhrzeiten der letzten Leerung und des nächsten automatischen Leerungsauftrages angegeben sind, ob Leerungsaufträge erteilt wurden, ob ggf. nicht lesbare Proben vorhanden sind einschließlich der Anzahl der nicht lesbaren Proben und der letzten Leerung der nicht lesbaren Proben sowie Warnmeldungen und Informationen über den technischen Gerätestatus enthalten.

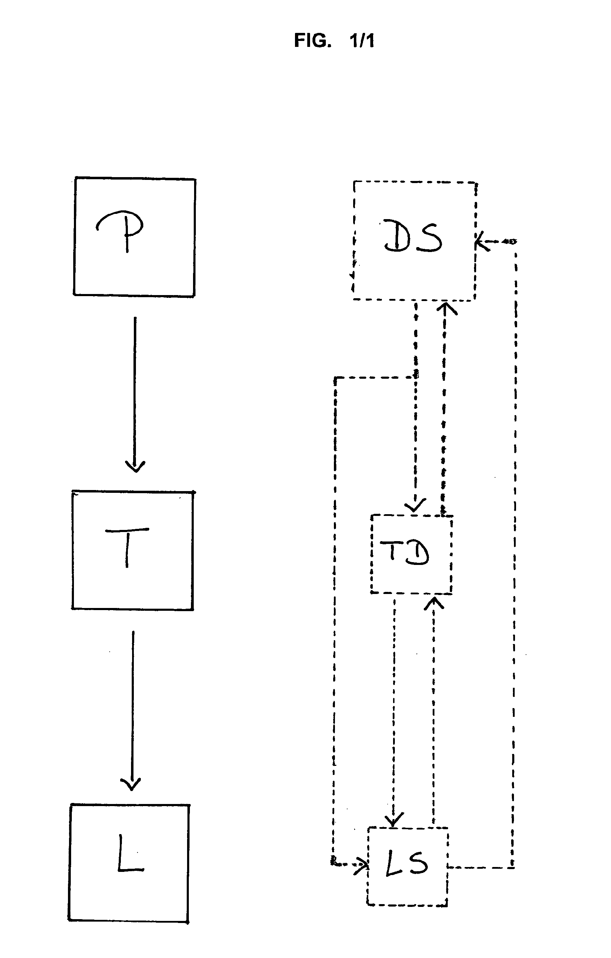

Die vorliegende Erfindung wird anhand der beigefügten Zeichnung näher erläutert. In der beigefügten Figur ist ein schematisches Ablaufdiagramm, das das erfindungsgemäße Verfahren erläutert, dargestellt.

Nach der Entscheidung ob und welche Untersuchung z. B. an einem Patienten durchgeführt werden sollen, wird diese zusammen mit allen notwendigen Daten als Datensatz in das Datensystem eingegeben. Erfolgt der Auftrag zur Probenentnahme nicht unmittelbar mündlich oder schriftlich, kann dieser über das Datensystem an die Person, die die Probe zu entnehmen hat, ergehen. Der Auftrag zur Probenentnahme enthält beispielsweise auch eine zeitliche Vorgabe, bis zu welchem Zeitpunkt die Proben entnommen werden sollten, diese zeitliche Vorgabe kann beispielsweise der Arbeitsauftrag für den nächsten Tag für Mitarbeiter sein, in einem Krankenhaus zum Beispiel der Auftrag des Arztes an das Pflegepersonal etc.

Mit der Erfassung der Daten für die durchzuführende Untersuchung in das Datensystem erfolgt ein Vorankündigung bzw. Beauftragung an das Labor, vorzugsweise über Datenleitungen. Das Labor bestätigt den angekündigten Untersuchungsauftrag und gibt als Rückmeldung, vorzugsweise über das Laborsystem berechnet und via Datenleitung, in welcher Form und Menge die Proben benötigt werden. Aus dieser Information erstellt das Datensystem des Auftraggebers bereits die entsprechenden Etiketten für die Anbringung am Proberöhrchen.

Nach der Probenabnahme wird die Probe, versehen mit der entsprechenden Kennzeichnung, in einen Probensammler gegeben. Sofern das Datensystem bereits alle für die durchzuführende Untersuchung erforderlichen Daten enthält, ist es möglich, über die im Probensammler befindlichen Proben abzugleichen, ob ausreichend Probenmaterial entnommen wurde. Auch der Status, dass die Probe P sich inzwischen im Probensammler befindet, kann über das Datensystem abgefragt werden.

Das Probenüberwachungssystem PS wird beispielsweise in einem Krankenhaus vom Stationspersonal bedient, entsprechende Eingabestationen sollten leicht erreichbar sein, beispielsweise in einer zentralen Position innerhalb der Station. In der Umweltanalytik ist bevorzugt ein transportables Datensystem zu verwenden und die entsprechenden Daten direkt vor Ort einzugeben.

Der Transport der Probe P zum Labor erfolgt in einem dafür vorgesehenen Transportbehälter.

Vom Probenüberwachungssystem oder einem davon getrennten System für die Verwaltung des Probentransports wird eine Auflistung der darin enthaltenen Proben, d.h. Leerungsaufträge, erzeugt. Der Leerungsauftrag selbst wird nach bestimmten vom Datensystem oder vom Transportsystem vorgegebenen Bedingungen gegeben, nämlich wenn ein Notfallauftrag vorliegt und/oder Notfallproben vollständig im Datensystem erfasst sind und schon im Transportbehälter abgelegt sind, die älteste Probe über einen Zeitraum von xx Minuten erfasst ist, wobei xx je nach Sammelstelle, Tageszeit und Kalenderdatum konfiguriert ist, oder der Transportbehälter zu einem vorgegebenen Anteil yy Prozent gefüllt ist, wobei yy wiederum nach Sammelstelle, Tageszeit und Kalenderdatum konfigurierbar ist. Es ist auch möglich, in Einzelfällen den Leerungsauftrag manuell auszulösen oder eine spontane Leerung elektronisch zu dokumentieren. Sobald der Leerungsauftrag ergangen ist und die Proben sich auf dem Weg zum Labor befinden, ergeht eine Meldung vom Transportdienst TD zum Datensystem der Station. Im Datensystem DS der Station wird vermerkt, dass die Probe bzw. der Transportbehälter abgeholt wurde. Der Probenstatus ändert sich auf "Probe auf dem Weg ins Labor".

Im Labor wird die Probe erneut identifiziert, d.h. sowohl der Transportbehälter als auch die Proben selbst werden erfasst und vorzugsweise auf ihre Unversehrtheit hin überprüft.

In der Praxis verfährt der Transportdienst derart, dass dieser sich gegenüber der Probe, d.h. dem Transportbehälter als berechtigter Abholer identifiziert. Vorzugsweise wird der abgeholte Transportbehälter durch einen leeren ersetzt.

Bei Ankunft der Probe im Labor L wird diese vom Laborsystem LS erfasst. Die eingegangenen Proben werden mit der vom Datensystem DS bzw. vom Transportdienst TD gemeldeten Proben abgeglichen. Die Ankunft der Proben im Labor L wird vom Laborsystem LS dem Transportdienst TD und dem Datensystem DS des Auftraggebers gemeldet. Im Labor werden die Proben anschließend vorbereitet und die Untersuchung wird durchgeführt. Die Untersuchungsergebnisse werden nach Ihrer Freigabe an das Datensystem DS des Auftraggebers gemeldet.

## Patentansprüche

1. Verfahren zur Überwachung von zu untersuchenden Proben, die nach der Probenentnahme an einen vom Ort der Probenentnahme verschiedenen Ort untersucht werden, welches die folgenden Schritte umfasst:
a) Erfassen der Probe in dem Datensystem (DS) des Auftraggebers,
b) Generierung eines Laborauftrages aus den Daten über vorzunehmende Analysen/Untersuchungen,
c) Erstellung eines probenbezogenen Datensatzes im Datensystem (DS) des Auftraggebers und Übermittlung des Datensatzes an das Laborsystem (LS) des beauftragten Labors sowie an das Probenüberwachungssystem,
d) Zusammenstellen der für das beauftragte Labor bestimmten Proben zu mindestens einer Charge, im Datensystem (DS) des Auftraggebers Generierung eines für die Charge spezifischen Chargen-Identifizierungscodes, z.B. einer Fallnummer, sowie Zuordnung der Datensätze der einzelnen Proben der Charge zu dem Chargen-Identifizierungscode,
e) i. ggf. Generierung eines Transportauftrags an einen Transportdienst, ii. Erfassung der Abholung der Probe(n) bzw. Charge(n) im Probenüberwachungssystem durch einen Transportdienst und Verknüpfung des Auftrages mit den Chargen-Identifizierungscodes der zu transportierenden Chargen,
f) Transport der Proben an das beauftragte Labor,
g) per Datentransfer an das Datensystem (DS) des Auftraggebers Bestätigung über den Probeneingang im beauftragten Labor anhand des Chargen-Identifizierungscodes und/oder der Datensätze der einzelnen Proben,
h) Durchführung der Untersuchung,
i) per Datentransfer an das Datensystem (DS) des Auftraggebers Übermittlung der mit dem jeweiligen Datensatz verknüpften Untersuchungsergebnisse.

2. Verfahren nach Anspruch 1,
**gekennzeichnet durch**
dass die Probe mit einem Probenidentifizierungscode versehen wird.

3. Verfahren nach Anspruch 1 oder 2,
**gekennzeichnet durch**
datenmäßige Verknüpfung des Datensatzes und/oder des Proben-Identifizierungscodes mit einer Dringlichkeitsstufe.

4. Verfahren nach einem der Ansprüche 1 bis 3,
**dadurch gekennzeichnet,**
**dass** die Probe mit einer Dringlichkeitsstufe klassifiziert wird. Verfahren nach einem der Ansprüche 1 bis 3, **dadurch gekennzeichnet, dass** die Proben in einem Probensammler abgelegt, identifiziert und spätestens nach einer vorbestimmten Zeit xx zum Labor gebracht werden.

5. Verfahren nach Anspruch 4, **dadurch gekennzeichnet, dass** die Zeit xx von der Dringlichkeitsstufe der Probe abhängt.

6. Verfahren nach einem der Ansprüche 1 bis 5, **dadurch gekennzeichnet, dass** der aktuelle Aufenthaltsort der Probe und der Status der Probe vom Probenüberwachungssystem erfasst wird.

7. Verfahren nach einem der Ansprüche 1 bis 6, **dadurch gekennzeichnet, dass** vom Datensystem nicht erfassbare Proben in eine für diese Proben vorgesehene Vorrichtung gegeben werden.

8. Verfahren nach einem der Ansprüche 1 bis 7, **dadurch gekennzeichnet, dass** der Probensammler (Transportbehälter) mit einem Identifizierungscode versehen ist, welcher als Anzeige bzw. zur Identifizierung für den Transportdienst dient.

9. Verfahren nach einem der Ansprüche 1 bis 8, **dadurch gekennzeichnet, dass** vom Transportdienst beim Abholen der Probe eine Meldung an das Datensystem DS sowie an das Labor ergeht.

10. Verfahren nach einem der Ansprüche 1 bis 8, **dadurch gekennzeichnet, dass** die Proben Patientenproben eines Krankenhauses sind.
